(19) **Europäisches Patentamt / European Patent Office / Office européen des brevets**

(11) **EP 3 239 688 B1**

(12) **EUROPEAN PATENT SPECIFICATION**

(45) Date of publication and mention of the grant of the patent:
**11.03.2020 Bulletin 2020/11**

(21) Application number: **15872139.9**

(22) Date of filing: **16.11.2015**

(51) Int Cl.:
*G01N 1/22* (2006.01)      *B01D 53/02* (2006.01)
*B01D 53/04* (2006.01)      *B01J 20/02* (2006.01)
*B01J 20/20* (2006.01)      *B01J 20/28* (2006.01)
*B82Y 30/00* (2011.01)      *G01N 1/32* (2006.01)
*G01N 27/04* (2006.01)      *G01N 27/12* (2006.01)

(86) International application number:
**PCT/JP2015/005691**

(87) International publication number:
**WO 2016/103561 (30.06.2016 Gazette 2016/26)**

(54) **CHEMICAL SUBSTANCE CONCENTRATOR AND CHEMICAL SUBSTANCE DETECTING DEVICE**

KONZENTRATOR EINER CHEMISCHEN SUBSTANZ UND VORRICHTUNG ZUR DETEKTION EINER CHEMISCHEN SUBSTANZ

CONCENTRATEUR DE SUBSTANCE CHIMIQUE ET DISPOSITIF DE DÉTECTION DE SUBSTANCE CHIMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2014 JP 2014258325**

(43) Date of publication of application:
**01.11.2017 Bulletin 2017/44**

(73) Proprietor: **Panasonic Intellectual Property Management Co., Ltd.**
**Osaka-shi, Osaka 540-6207 (JP)**

(72) Inventors:
• **NAKAO, Atsuo**
**Osaka-shi, Osaka 540-6207 (JP)**
• **OKA, Hiroaki**
**Osaka-shi, Osaka 540-6207 (JP)**

• **YANAGIDA, Takeshi**
**Osaka-shi, Osaka 540-6207 (JP)**

(74) Representative: **Grünecker Patent- und Rechtsanwälte**
**PartG mbB**
**Leopoldstraße 4**
**80802 München (DE)**

(56) References cited:
**EP-A1- 3 351 920          WO-A1-01/87461**
**WO-A1-03/051490      WO-A1-2009/147294**
**JP-A- 2012 516 991      JP-A- 2014 504 740**
**US-A1- 2009 282 899    US-A1- 2009 317 916**
**US-A1- 2010 255 597    US-A1- 2013 018 599**

• **HYANG HEE CHOI: 'Noxious gas detection using carbon nanotubes with Pd nanoparticles' NANOSCALE RESEARCH LETTERS vol. 6, no. 605, 24 November 2011, pages 1 - 6, XP055393868**

**Description**

TECHNICAL FIELD

The present invention relates to a technique for analyzing and detecting a chemical substance in a gas.

BACKGROUND ART

As a technique for analyzing a chemical substance in a gas, PTL 1 discloses a device for analyzing an organic substance in a gas inside electric power equipment. In this device, a gas passes through a pipe while a temperature of a trap is constant so that an organic substance in the gas is adsorbed on an adsorbent. Then, the trap is heated to introduce the adsorbed organic substance to a detector. PTL 2 discloses a device for trace level detection of an analyte using an adsorbent material capable of adsorbing an analyte and desorbing a concentrated analyte.

WO 2009/147294 A1 relates to an equipment for concentrating and analyzing components contained in a flowing medium, the analyzing equipment comprising a concentrator including a porous sorbent bed, in which components are absorbed or adsorbed from the medium flow, said bed being electroconductive. WO 03/051490 A1 relates to removing of contaminant from a gaseous stream by adsorption on a sorbent which is an activated carbon cloth material. US 2010/255597 A1 relates to a room temperature CO sensor and method of making the same. WO 01/87461 A1 relates to an adsorption/desorption unit including a hollow enclosure containing one or more elongate hollow elements of activated carbon fiber cloth. JP 2014 504740 A relates to a chemical preconcentrator including a conduit defining a flow path between two ends and a heating element disposed within the conduit. US 2013/018599 A1 relates to a graphene nano-sensor with a suspended graphene flake electrically connected to metal electrodes.

CITATION LIST

PATENT LITERATURES

PTL 1: Japanese Patent Laid-Open Publication No. 2001-296218
PTL 2: Japanese Patent Laid-Open Publication No. 2002-518668

SUMMARY

A chemical substance concentrator according to the invention, configured to concentrate a chemical substance in a gaseous object, is defined in claim 1. Further advantageous embodiments are defined in the dependent claims.
The chemical substance concentrator can desorb the adsorbed chemical substance with low power consumption.

BRIEF DESCRIPTION OF DRAWINGS

FIG. 1 is a schematic diagram of a chemical substance concentrator according to an exemplary embodiment of the invention.
FIG. 2 is a schematic diagram of a detecting device including the chemical substance concentrator according to the embodiment of the invention.
FIG. 3A illustrates an arrangement of a conductive adsorbent in a channel of the chemical substance concentrator according to an example not part of the invention but facilitates understanding of the invention.
FIG. 3B illustrates another arrangement of the conductive adsorbent in the channel of the chemical substance concentrator according to the
embodiment of the invention.
FIG. 3C illustrates still another arrangement of the conductive adsorbent in the channel of the chemical substance concentrator according to the embodiment of the invention.

DETAIL DESCRIPTION OF PREFERRED EMBODIMENT

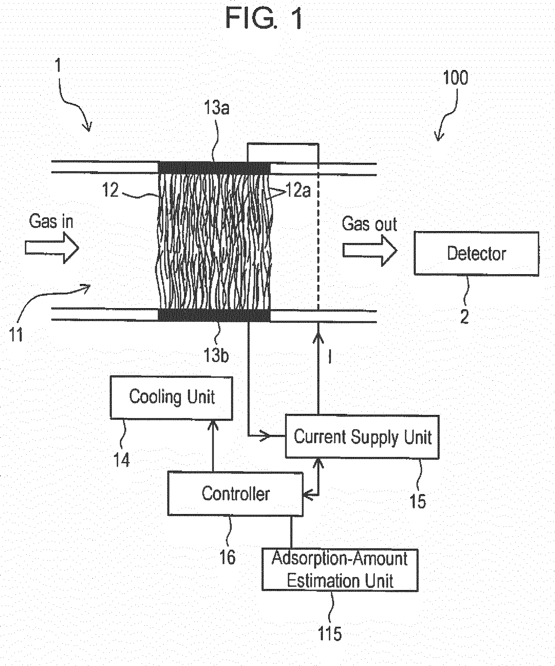

FIG. 1 is a schematic diagram of chemical substance concentrator 1 of detecting device 100 according to an exemplary embodiment. In chemical substance concentrator 1 (hereinafter referred to as a concentrator), adsorbent 12 adsorbs a chemical substance in a gaseous object flowing into concentrator 1, concentrates the adsorbed chemical substance, then desorbs the chemical substance from the adsorbent by heating, and sends the chemical substance to detector 2 at a subsequent stage. The chemical substance may be volatile organic compounds, such as ketones, amines, alcohols, aromatic hydrocarbons, aldehydes, esters, organic acids, hydrogen sulfide, methyl mercaptan, and disulfide.

Concentrator 1 includes channel 11 in which a gaseous object flows, conductive adsorbent 12 disposed in channel 11, a pair of electrodes 13a and 13b configured to cause a current to flow in adsorbent 12, and cooling unit 14 configured to cool the gaseous object flowing in channel 11. Current supply unit 15 supplies current I to the pair of electrodes 13a and 13b. Controller 16 controls operations of cooling unit 14 and current supply unit 15. Adsorbent 12 is configured to adsorb a chemical substance contained in the gaseous object.

Concentrator 1 according to the embodiment includes conductive nanowires 12a connected between electrodes 13a and 13b facing each other as adsorbent 12 for a chemical substance. That is, in a process of cooling by cooling unit 14, the chemical substance in the gaseous object is adsorbed on surfaces of nanowires 12a, and is collected and concentrated. Then, current supply unit 15 causes a slight amount of current I to flow in nanowires 12a through electrodes 13a and 13b so that nanowire 12a generates heat (self-heating) by the Joule effect. A temperature rise due to the self-heating of nanowires 12a causes the chemical substance adsorbed on the surface of nanowire 12a to be desorbed and introduced to detector 2 at the subsequent stage. That is, conductive nanowires 12a function also as a heating unit for heating the chemical substance.

This configuration and operation of concentrator 1 can send the concentrated chemical substance to detector 2 with low power consumption without using an external heater consuming a large amount of electric power.

In conventional configurations disclosed in PTLs 1 and 2 requires a heating unit, such as an external heater, in introducing an adsorbed chemical substance to a detector. If a chemical substance is desorbed without using a heater, the chemical substance is insufficiently desorbed. For this reason, the conventional detecting devices consume a large amount of electric power. Specifically, a heater used in the conventional technique needs about several tens to several hundreds of milliwatts of electric power. A technique for micro electro mechanical systems (MEMS) includes, e.g. a Pt-wire resistance heater requiring several milliwatts or more of power consumption.

As described above, a heater used in the conventional technique requires about several tens to several hundreds of milliwatts of electric power, whereas the technique according to this embodiment can desorb the chemical substance with electric power not larger than about $10\mu$W. In addition, the technique according to the embodiment needs no external heaters, hence reducing the size of detecting device 100 including the concentrator.

Here, when current I flows in a conductor, heat quantity Q generated by resistance R of the conductor is expressed as follows (Joule effect):

$$Q = R \cdot I^2 \cdot t$$

A relation between heat quantity Q and temperature change $\Delta T$ can be expressed with thermal capacity C of a conductor that is a product of a specific heat and a mass of the conductor:

$$Q = C \cdot \Delta T$$

Thermal capacity C depends on the mass (volume) of the conductor. Thus, a substance, such as nanowire 12a, having a slight volume has small thermal capacity C. A very small amount of heat quantity Q generated when a current flows provides a large amount of temperature change $\Delta T$.

In accordance with this embodiment, the reason for using nanowires 12a as adsorbent 12 is that nanowires 12a provide the absorbent with a large specific surface area and a high concentration (adsorption) efficiency, and in addition, has small thermal capacity C providing a large temperature change with low power consumption. Another material having a large specific surface area may be a porous body. The porous body, however, has a larger volume than nanowires 12a, and consumes larger electric power than nanowires 12a due to self-heating by the Joule effect. Conductive adsorbent 12 according to this embodiment may not necessarily be implemented by nanowires 12a, but by another structure, such as the porous body.

Conductive adsorbent 12, such as nanowires 12a or the porous body, contains metal oxide, such as $SnO_2$, ZnO, $In_2O_3$, $In_{2-x}Sn_xO_3$ (where $0.1 \leq x \leq 0.2$, for example), NiO, CuO, $TiO_2$, or $SiO_2$, metal, such as Al, Ag, Au, Pd, and Pt, and conductive material, such as carbon or silicon. As nanowires of carbon, carbon nanotubes may be used, for example. That is, a material of the adsorbent is made of a conductive material having a resistance enough to effectively exhibit self-heating due to the Joule effect.

The material of electrodes 13a and 13b according to this embodiment may be metal, such as gold, platinum, silver, copper, or aluminium, conductive oxide, such as indium tin oxide (ITO) or Al-doped zinc oxide (AZO), or conductive polymers. Electrodes 13a and 13b may have uneven surfaces. That is, electrodes 13a and 13b have shapes conforming with unevenness of the surface of nanowires 12a or the porous body. Apart of adsorbent 12 may be embedded in electrodes 13a and 13b. For example, respective ends of nanowires 12a may be embedded in electrodes 13a and 13b. A part of the porous body may be embedded in electrodes 13a and 13b. Chemical substance concentrator 1 can thereby connect electrically between adsorbent 12 and each of electrode 13a and 13b.

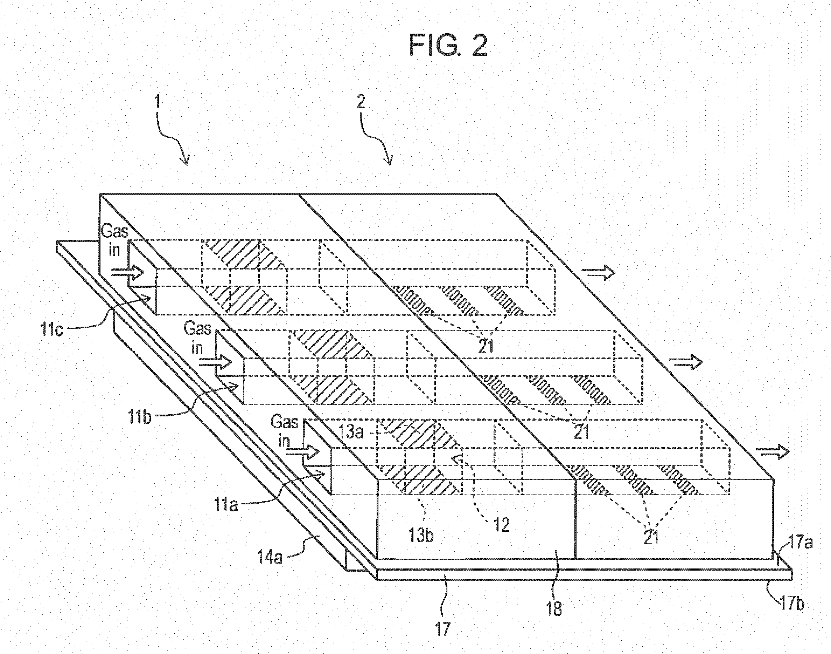

FIG. 2 is a schematic diagram of chemical substance detecting device 100 including concentrator 1 according to the embodiment. In the configuration illustrated in FIG. 2, frame 18 made of, e.g. polydimethylsiloxane (PDMS), epoxy resin, polyvinylidene chloride resin, or glass is provided on surface 17a of substrate 17, such as a silicon substrate, a glass epoxy substrate, or a ceramics substrate. Channels 11a, 11b, and 11c are formed in frame 18 as microchannels in which a gaseous object flows. Channels 11a, 11b, and 11c extend to detector 2 from concentrator 1 into which the gaseous object flows. Although three channels 11a, 11b, and 11c are shown in FIG. 2, the number of channels is not limited to three.

In concentrator 1, each pair of electrodes 13a and 13b are disposed on top and bottom of respective one of channels 11a, 11b, and 11c. Nanowires 12a are disposed between electrodes 13a and 13b. Cooling device 14a, such as a Peltier device, is provided on surface 17b of substrate 17 opposite to surface 17a of substrate 17. Cooling device 14a functions as cooling unit 14 configured to cool a gaseous object flowing in channels 11a, 11b, and 11c. In detector 2, sensors 21 for detecting a particular chemical substance are arranged in channels 11a, 11b, and 11c. In FIG. 2, wires for allowing current to flow in electrodes 13a and 13b, wires for supplying electric power to sensors 21, and wires for outputting detection signals of sensors 21 are not shown.

The configuration illustrated in FIG. 2 of concentrator 1 adsorbs and concentrates the chemical substance contained in the gaseous object flowing in channels 11a, 11b, and 11c, and detector 2 detects the concentrated chemical substance. That is, in a process of cooling by cooling device 14a, the chemical substance in the gaseous object is adsorbed on the surfaces of nanowires 12a, and is concentrated and collected. Then, a current flows in nanowires 12a through electrodes 13a and 13b, thereby causing self-heating of nanowire 12a. A temperature rise due to the self-heating causes the chemical substance adsorbed on the surface of nanowire 12a to be desorbed, introduced to detector 2, and detected by sensors 21.

In the configuration illustrated in FIG. 2, electrodes 13a and 13b are disposed on top and bottom of each of channels 11a, 11b, and 11c. However, the positions of electrodes 13a and 13b are not limited to this configuration, and electrodes 13a and 13b may be disposed at any locations as long as current can flow in nanowire 12a.

In the configuration illustrated in FIG. 2, cooling device 14a is disposed on surface 17b of substrate 17. The position of cooling device 14a is not limited to this configuration, and may be at any location as long as cooling device 14a can cool a gaseous object. For example, cooling device 14a may be disposed on frame 18 or on electrode 13a or 13b. In the case where cooling device 14a is disposed on electrode 13a or 13b, an insulator may be disposed between cooling device 14a and electrode 13a or 13b.

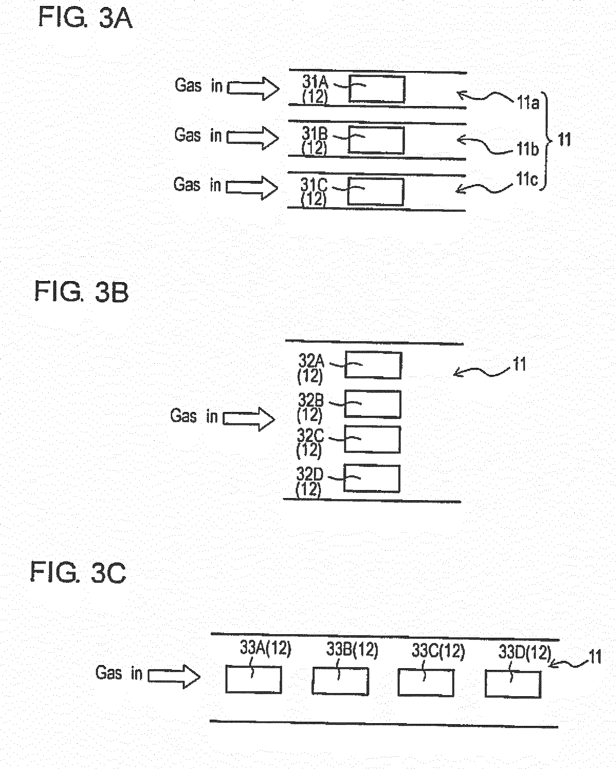

FIGS. 3A to 3C illustrate arrangements of conductive adsorbent 12, such as nanowires 12a. In FIG. 3A, adsorbent 12 includes plural groups 31A to 31C that are disposed separately from one another. Similarly to the configuration illustrated in FIG. 2, groups 31A, 31B, and 31C are disposed in channels 11a, 11b, and 11c, respectively. In FIG. 3B, adsorbent 12 includes plural groups 32A to 32D that are disposed separately from one another, and groups 32A, 32B, 32C, and 32D are aligned perpendicularly to a direction in which the gaseous object flows in channel 11. In FIG. 3C, adsorbent 12 includes plural groups 33A to 33D that are disposed separately from one another, and groups 33A, 33B, 33C, and 33D are aligned in a direction in which the gaseous object flows in channel 11.

As illustrated in FIG. 2 and FIGS. 3A to 3C, conductive adsorbent 12 (nanowires 12a) includes plural groups separate from one another. In this case, the groups constituting conductive adsorbent 12 is made of different materials or may be provided with different surface modifications (coatings). This configuration enables gas molecules to be selectively adsorbed and concentrated. For example, since gas molecules are easily adsorbed on the surface having the same polarity as the gas molecules, if plural nanowires 12a (adsorbents 12) having surfaces having different polarities are prepared as the conductive adsorbents, adsorption of polar molecules is dominant over adsorption of non-polar molecules on conductive adsorbent 12 having a surface having high polarity, and adsorption of non-polar molecules is dominant over adsorption of polar molecules on the conductive adsorbent having a surface having no polarity. That is, the groups of conductive adsorbent 12 preferably include groups made of different materials. Alternatively, the groups of adsorbent 12 preferably include groups provided with different surface modifications.

In the case where conductive adsorbent 12 includes plural groups, current supply unit 15 for supplying a current to conductive adsorbent 12 is preferably configured to selectively supply currents to plural pairs of electrodes provided on the groups. In this manner, the timing of desorbing the chemical substance adsorbed on each group of conductive adsorbent 12 can be controlled for each group of conductive adsorbent 12. For example, chemical substances adsorbed on the groups of conductive adsorbent 12 can be sent in a predetermined order to a detector at a subsequent stage. Thus, in the case where the chemical substance adsorbed on each group of conductive adsorbent 12 is specified, a precise analyzer is not needed as the detector at a subsequent stage. As a result, the size of detecting device 100 can be reduced.

In detecting device 100 according to the embodiment, since a current flows from current supply unit 15 to conductive adsorbent 12, a resistance of conductive adsorbent 12 can be monitored. On the other hand, the resistance of conductive adsorbent 12 changes depending on adsorption of the chemical substance. For example, in the case where conductive adsorbent 12 is made of metal oxide, the amount of oxygen in the surface of conductive adsorbent 12 changes depending on adsorption of the chemical substance to change the resistance. Even in the case where conductive adsorbent 12 is made of silicon, as long as adsorbed molecules have polarity, the resistance changes in accordance with the amount of adsorption of the molecules. Thus, as illustrated in FIG. 1, concentrator 1 according to the embodiment may include adsorption-amount estimation unit 115 that estimates the amount of the chemical substance adsorbed on conductive adsorbent 12 based on the change of the resistance of conductive adsorbent 12.

Adsorption-amount estimation unit 115 previously stores a relation between the amount of adsorption of a chemical substance and a change of a resistance of nanowires 12a of adsorbent 12. Adsorption-amount estimation unit 115 detects the change of the resistance value based on the amount of the current flowing in adsorbent 12. Based on the detected change, the amount of a chemical substance adsorbed on adsorbent 12 is estimated with reference to the previously stored relation between the resistance and the amount of adsorption of the chemical substance. The presence of adsorption-amount estimation unit 115 as described above enables controller 16 to more accurately control a timing when an adsorbed chemical substance is desorbed.

As described above, chemical substance concentrator 1 is configured to concentrate a chemical substance in a gaseous object. Chemical substance concentrator 1 includes channel 11 (11a, 11b, 11c) in which the gaseous object flows, conductive adsorbent 12 that is disposed in channel 11 (11a, 11b, 11c) and adsorbs the chemical substance, the pair of electrodes 13a and 13b for causing current to flow in adsorbent 12, and cooling unit 14 for cooling the gaseous object flowing in channel 11 (11a, 11b, 11c).

In this aspect, conductive adsorbent 12 that adsorbs the chemical substance is disposed in channel 11 in which the gaseous object flows. The pair of electrodes 13a and 13b configured to cause a current to flow in adsorbent 12. In a process of cooling by cooling unit 14, the chemical substance in the gaseous object is adsorbed on the surface of adsorbent 12, and is concentrated and collected. Then, a current flows in adsorbent 12 through electrodes 13a and 13b so that adsorbent 12 can generate heat due to the Joule effect. A temperature rise due to this heat causes the chemical substance adsorbed on the surface of adsorbent 12 to be desorbed. Chemical substance concentrator 1 can thus send the concentrated chemical substance to detector 2 with low power consumption without using an external heater consuming a large amount of electric power. In addition, since no external heaters are needed, the size of detecting device 100 can be reduced.

In the case where a sufficient amount of the chemical substance is adsorbed on adsorbent 12 without cooling the gaseous object, chemical substance concentrator 1 may not necessarily include cooling unit 14 (not according to the invention).

Adsorbent 12 may be nanowires 12a.

Nanowires 12a can cause a large temperature change with low power consumption so that power consumption of chemical substance concentrator 1 can be further reduced.

Adsorbent 12 may be a porous body.

Adsorbent 12 may contain a metal oxide, a metal, carbon, or silicon.

Adsorbent 12 includes groups 31A to 31C that are disposed separately from one another.

Groups 31A to 31C of adsorbent 12 include groups made of different materials or may be provided with different surface modifications.

In these aspects, groups 31A to 31C of adsorbent 12 can selectively concentrate different types of chemical substances.

Chemical substance concentrator 1 may further include current supply unit 15 configured to supply a current to the pair of electrodes 13a and 13b. The pair of electrodes 13a and 13b includes plural pairs of electrodes 13a and 13b each provided on respective one of groups 31A to 31C of adsorbent 12. Current supply unit 15 is configured to selectively supply currents to the pairs of electrodes 13a and 13b.

In this aspect, the adsorbed and concentrated chemical substance can be selectively desorbed from adsorbent 12.

Chemical substance concentrator 1 may include substrate 17 having surfaces 17a and surface 17b opposite to surface 17a, and frame 18 provided on surface 17a of substrate 17. Frame 18 includes channel 11 (11a, 11b, 11c) therein. Cooling unit 14 is cooling device 14a disposed on surface 17b of substrate 17.

Adsorption-amount estimation unit 115 detects a change of a resistance of adsorbent 12 based on an amount of a current flowing in adsorbent 12, and estimates, based on the detected change, an amount of the chemical substance adsorbed on adsorbent 12.

In this aspect, timing when the adsorbed and concentrated chemical substance is desorbed can be more accurately controlled.

Detecting device 100 includes chemical substance concentrator 1 and detector 2 into which the chemical substance concentrated by chemical substance concentrator 1 is introduced. Detector 2 includes a detection element, such as a semiconductor sensor, an electrochemical sensor, an elastic wave sensor, or a field effect transistor sensor. The detection element is not limited to these sensors. Detector 2 can employ an optimum detection element for detecting the chemical substance concentrated by chemical substance concentrator 1.

The above configuration provides chemical substance detecting device 100 (100A) with low power consumption and a small size.

INDUSTRIAL APPLICABILITY

A chemical substance concentrator according to the present invention can detect a chemical substance in a gaseous object with a small-size detecting device with low power consumption, and thus, is useful for, e.g. an ultrasmall chemical sensor capable of detecting a volatile organic compound in user's environments.

REFERENCE MARKS IN THE DRAWINGS

| | |
|---|---|
| 1 | chemical substance concentrator |
| 2 | detector |
| 11, 11a, 11b, 11c | channels |
| 12 | adsorbent |
| 12a | nanowire |
| 13a, 13b | electrodes |
| 14 | cooling unit |
| 14a | cooling device |
| 15 | current supply unit |
| 16 | controller |
| 17 | substrate |
| 18 | frame |
| 31A-31C, 32A-32D, 33A-33D | adsorbent groups |
| 100 | detecting device |
| 115 | adsorption-amount estimation unit |

**Claims**

1. A chemical substance concentrator configured to concentrate a chemical substance in a gaseous object, the chemical substance concentrator comprising:

   a channel (11) in which the gaseous object flows;
   an adsorbent (12) disposed in the channel (11), the adsorbent (12) being conductive and configured to adsorb the chemical substance; and
   a pair of electrodes (13a, 13b) configured to cause a current to flow in the adsorbent (12),
   **characterized in that**
   the adsorbent (12) comprises a plurality of groups of adsorbents (32A-32D, 33A-33D) disposed separately from one another, and
   the plurality of groups of adsorbents (32A-32D, 33A-33D) of the adsorbent comprises groups made of different materials or groups having different surface modifications thereon,
   the chemical substance concentrator further comprises a cooling unit (14) configured to cool the gaseous object flowing in the channel (11).

2. The chemical substance concentrator of claim 1, wherein the adsorbent (12) comprises nanowires.

3. The chemical substance concentrator of claim 1, wherein the adsorbent (12) comprises a porous body.

4. The chemical substance concentrator of any one of claims 1 to 3, wherein the adsorbent (12) contains metal oxide, metal, carbon, or silicon.

5. The chemical substance concentrator of claim 1, further comprising
a current supply unit (15) configured to supply a current to the pair of electrodes (13a, 13b),
wherein the pair of electrodes (13a, 13b) comprises a plurality of pairs of electrodes, each of the pairs of electrodes being provided on respective one of the plurality of groups of adsorbents (32A-32D, 33A-33D) of the adsorbent (12), and
wherein the current supply unit (15) is configured to supply the current selectively to the plurality of pairs of electrodes.

6. The chemical substance concentrator of claim 1, further comprising:

   a substrate (17) having a first surface (17a) and a second surface (17b) opposite to the first surface (17a); and
   a frame (18) disposed on the first surface (17a) of the substrate (17), the frame (18) having the channel (11a, 11b, 11c) therein,
   wherein the cooling unit (14) is a cooling device (14a) disposed on the second surface (17b) of the substrate (17).

7. The chemical substance concentrator of claim 1, further comprising an adsorption-amount estimation unit (115) configured to detect a change of a resistance of the adsorbent (12) based on an amount of a current flowing in the adsorbent (12) as to estimates an amount of the chemical substance adsorbed on the adsorbent (12) based on the change.

8. A chemical substance detecting device comprising:

   the chemical substance concentrator (1) of claim 1; and
   a detector (2, 21) to which the chemical substance concentrated by the chemical substance concentrator (1) is introduced.

**Patentansprüche**

1. Vorrichtung zum Konzentrieren einer chemischen Substanz, die zum Konzentrieren einer chemischen Substanz in einem gasförmigen Objekt ausgeführt ist, wobei die Vorrichtung zum Konzentrieren einer chemischen Substanz umfasst:

   einen Kanal (11), in dem das gasförmige Objekt strömt;
   ein Adsorptionsmittel (12), das sich in dem Kanal (11) befindet, wobei das Adsorptionsmittel (12) leitfähig und zum Adsorbieren der chemischen Substanz ausgeführt ist; sowie
   paarige Elektroden (13a, 13b), die so ausgeführt sind, dass sie einen Stromfluss in dem Adsorptionsmittel (12) bewirken,
   **dadurch gekennzeichnet, dass**
   das Adsorptionsmittel (12) eine Vielzahl von Gruppen von Adsorptionsmitteln (32A-32D, 33A-33D) umfasst, die getrennt voneinander angeordnet sind, und
   die Vielzahl von Gruppen von Adsorptionsmitteln (32A-32D, 33A-33D) des Adsorptionsmittels Gruppen, die aus unterschiedlichen Materialien bestehen, oder Gruppen mit unterschiedlichen Oberflächenmodifikationen daran umfasst,
   die Vorrichtung zum Konzentrieren einer chemischen Substanz des Weiteren eine Kühl-Einheit (14) umfasst, die zum Kühlen des in dem Kanal (11) strömenden gasförmigen Objektes ausgeführt ist.

2. Vorrichtung zum Konzentrieren einer chemischen Substanz nach Anspruch 1, wobei das Adsorptionsmittel (12) Nanodrähte umfasst.

3. Vorrichtung zum Konzentrieren einer chemischen Substanz nach Anspruch 1, wobei das Adsorptionsmittel (12) einen porösen Körper umfasst.

4. Vorrichtung zum Konzentrieren einer chemischen Substanz nach einem der Ansprüche 1 bis 3, wobei das Adsorptionsmittel (12) Metalloxid, Metall, Kohlenstoff oder Silizium enthält.

**5.** Vorrichtung zum Konzentrieren einer chemischen Substanz nach Anspruch 1, die des Weiteren eine Stromzuführ-Einheit (15) umfasst, die zum Zuführen eines Stroms zu den paarigen Elektroden (13a, 13b) ausgeführt ist,

wobei die paarigen Elektroden (13a, 13b) eine Vielzahl von Paaren von Elektroden umfassen, wobei sich jedes der Paare von Elektroden an einer jeweiligen der Vielzahl von Gruppen von Adsorptionsmitteln (32A-32D, 33A-33D) des Adsorptionsmittels (12) befindet, und
die Stromzuführ-Einheit (15) so ausgeführt ist, dass sie den Strom selektiv der Vielzahl von Paaren von Elektroden zuführt.

**6.** Vorrichtung zum Konzentrieren einer chemischen Substanz nach Anspruch 1, die des Weiteren umfasst:

ein Substrat (17), das eine erste Fläche (17a) und eine der ersten Fläche (17a) gegenüberliegende zweite Fläche (17b) aufweist; sowie
einen Rahmen (18), der an der ersten Fläche (17a) des Substrats (17) angeordnet ist, wobei sich in dem Rahmen (18) der Kanal (11a, 11b, 11c) befindet,
und die Kühl-Einheit (14) eine Kühleinrichtung (14a) ist, die an der zweiten Fläche (17b) des Substrats (17) angeordnet ist.

**7.** Vorrichtung zum Konzentrieren einer chemischen Substanz nach Anspruch 1, die des Weiteren eine Einheit (115) zum Schätzen einer adsorbierten Menge umfasst, die so ausgeführt ist, dass sie eine Änderung eines Widerstands des Adsorptionsmittels (12) auf Basis einer Stärke eines in dem Adsorptionsmittel (12) fließenden Stroms erfasst und so eine Menge der an dem Adsorptionsmittel (12) adsorbierten chemischen Substanz auf Basis der Änderung schätzt.

**8.** Vorrichtung zum Erfassen einer chemischen Substanz, die umfasst:

die Vorrichtung (1) zum Konzentrieren einer chemischen Substanz nach Anspruch 1, sowie
einen Detektor (2, 21), in den die durch die Vorrichtung (1) zum Konzentrieren einer chemischen Substanz konzentrierte chemische Substanz eingeleitet wird.

**Revendications**

**1.** Concentrateur de substance chimique configuré pour concentrer une substance chimique dans un objet gazeux, le concentrateur de substance chimique comprenant :

un canal (11) dans lequel circule l'objet gazeux,
un adsorbant (12) disposé dans le canal (11), l'adsorbant étant conducteur et configuré pour adsorber la substance chimique, et
une paire d'électrodes (13a, 13b) configurées pour amener la circulation d'un courant dans l'adsorbant (12), **caractérisé en ce que**
l'adsorbant (12) comprend une pluralité de groupes d'adsorbants (32A à 32D, 33A à 33D) disposés séparément les uns par rapport aux autres, et
la pluralité de groupes d'adsorbants (32A à 32D, 33A à 33D) de l'adsorbant comprend des groupes constitués de matériaux différents ou des groupes présentant sur eux des modifications de surface différentes,
le concentrateur de substance chimique comprend en outre une unité de refroidissement (14) configurée pour refroidir l'objet gazeux circulant dans le canal (11).

**2.** Concentrateur de substance chimique selon la revendication 1, dans lequel l'adsorbant (12) comprend des nanofils.

**3.** Concentrateur de substance chimique selon la revendication 1, dans lequel l'adsorbant (12) comprend un corps poreux.

**4.** Concentrateur de substance chimique selon l'une quelconque des revendications 1 à 3, dans lequel l'adsorbant (12) contient un oxyde métallique, un métal, du carbone ou du silicium.

**5.** Concentrateur de substance chimique selon la revendication 1, comprenant en outre :

une unité de fourniture de courant (15) configurée pour délivrer un courant à la paire d'électrodes (13a, 13b),
dans lequel la paire d'électrodes (13a, 13b) comprend une pluralité de paires d'électrodes, chacune des paires d'électrodes étant disposée sur l'un respectif de la pluralité de groupes d'adsorbants (32A à 32D, 33A à 33D) de l'adsorbant (12), et
dans lequel l'unité de fourniture de courant (15) est configurée pour délivrer sélectivement le courant à la pluralité de paires d'électrodes.

6. Concentrateur de substance chimique selon la revendication 1, comprenant en outre :

un substrat (17) comportant une première surface (17a) et une seconde surface (17b) opposée à la première surface (17a), et
un châssis (18) disposé sur la première surface (17a) du substrat (17), le châssis (18) comportant en lui le canal (11a, 11b, 11c),
dans lequel l'unité de refroidissement (14) est un dispositif de refroidissement (14a) disposé sur la seconde surface (17b) du substrat (17).

7. Concentrateur de substance chimique selon la revendication 1, comprenant en outre une unité d'estimation de quantité d'adsorption (115) configurée pour détecter une variation de la valeur ohmique de l'adsorbant (12) sur la base de la quantité de courant circulant dans l'adsorbant (12) de sorte à estimer la quantité de substance chimique adsorbée sur l'adsorbant (12) sur la base de la variation.

8. Dispositif de détection de substance chimique comprenant :

le concentrateur de substance chimique (1) conforme à la revendication 1, et
un détecteur (2, 21) sur lequel est introduite la substance chimique concentrée par le concentrateur de substance chimique (1).

## FIG. 1

Gas in

Gas out

Detector

2

Cooling Unit

14

Current Supply Unit

15

Controller

16

Adsorption-Amount Estimation Unit

115

FIG. 2

# FIG. 3A

Gas in ⟹  31A
(12)                          ⟵  11a

Gas in ⟹  31B
(12)                          ⟵  11b  } 11

Gas in ⟹  31C
(12)                          ⟵  11c

# FIG. 3B

                              32A
                              (12)      ⟵  11

Gas in ⟹                     32B
                              (12)

                              32C
                              (12)

                              32D
                              (12)

# FIG. 3C

              33A(12)   33B(12)   33C(12)   33D(12)
Gas in ⟹                                              ⟵ 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009147294 A1
- WO 03051490 A1
- US 2010255597 A1
- WO 0187461 A1
- JP 2014504740 A
- US 2013018599 A1
- JP 2001296218 A
- JP 2002518668 A